# EUROPÄISCHE PATENTSCHRIFT

(11) **EP 0 653 412 B1**
(45) Veröffentlichungstag und Bekanntmachung des Hinweises auf die Patenterteilung: **21.01.1998**
(21) Anmeldenummer: 94117176.1
(22) Anmeldetag: 31.10.1994
(51) Int. Cl.: C07C 45/63, C07C 47/55

(54) **Verfahren zur Herstellung von 4,5-Difluorbenzaldehyden**
Process for preparing 4,5-difluorobenzaldehydes
Procédé pour la préparation de 4,5-difluorobenzaldéhydes

(30) Priorität: 11.11.1993 DE 4338525
(43) Veröffentlichungstag der Anmeldung: 17.05.1995
(73) Patentinhaber: BAYER AG, 51368 Leverkusen (DE)
(72) Erfinder: Marhold, Albrecht, Dr., D-51373 Leverkusen (DE)

(56) Entgegenhaltungen:
- EP-A- 0 289 942
- DE-A- 3 420 796

## Beschreibung

Die vorliegende Erfindung betrifft ein Verfahren zur Herstellung von kernfluorierten Benzaldehyden, die Fluoratome in 4- und 5-Stellung zur Aldehyd-Gruppe enthalten.

Aus der EP-B1-289 942 ist bekannt, daß man an chlorierten, bromierten und/oder jodierten Benzaldehyden die Halogenatome ganz oder teilweise gegen Fluor austauschen kann. Bei der Betrachtung der Beispiele dieser Patentschrift stellt man fest, daß man mit diesem Verfahren nur Chloratome, die sich in 2- und 4-Stellung zur Aldehydgruppe befinden, gegen Fluor austauschen kann (siehe Beispiele 1 bis 18). Chloratome in 3- und/oder 5-Stellung zur Aldehydgruppe verbleiben unverändert (siehe Beispiel 19). Wenn man also 3- und/oder 5-Fluorbenzaldehyde herstellen will, muß man einen grundsätzlich anderen Syntheseweg suchen.

Außerdem zeigt die EP-B1-289 942, daß man auf die dort beschriebene Weise 2,4-Dichlorbenzaldehyde nur in 2,4-Difluorbenzaldehyde, nicht jedoch in 2-Chlor-4-fluor- oder 2-Fluor-4-chlorbenzaldehyde überführen kann (Beispiel 16).

Es wurde nun ein Verfahren zur Herstellung von 4,5-Difluorbenzaldehyden der Formel (I) gefunden 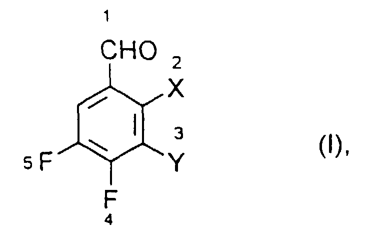 in der
- X: für Fluor oder Chlor und
- Y: für Wasserstoff oder Chlor stehen,
das dadurch gekennzeichnet ist, daß man Benzaldehyde der Formel (II) 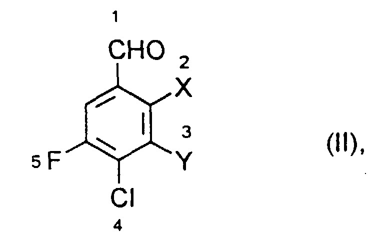 in der X und Y die bei Formel (I) angegebene Bedeutung haben, mit einem Fluorierungsmittel umsetzt.

Zum Einsatz in das erfindungsgemäße Verfahren sind beispielsweise 2,5-Difluor-4-chlorbenzaldehyd, 5-Fluor-2,4-chlorbenzaldehyd, 3,5-Difluor-3,4-dichlorbenzaldehyd und 5-Fluor-2,3,4-trichlorbenzaldehyd geeignet.

Als Fluorierungsmittel kommen beispielsweise Natrium-, Kalium- und/oder Cäsiumfluorid in Frage. Bevorzugt ist Kaliumfluorid und eine Mischung aus Natriumfluorid und Kaliumfluorid sowie eine Mischung aus Natriumfluorid und Cäsiumfluorid.

Bezogen auf 1 mol auszutauschenden Chlors im eingesetzten Benzaldehyd der Formel (II) kann man beispielsweise 0,8 bis 2 mol Fluorierungsmittel einsetzen Vorzugsweise liegt diese Menge bei 1 bis 1,5 mol.

Es ist im allgemeinen vorteilhaft, die erfindungsgemäße Fluorierung in Gegenwart eines Lösungsmittels durchzuführen. Als Lösungsmittel kommen beispielsweise in Frage: Dimethylacetamid, Diethylacetamid, Dibutylacetamid, N-Metylpyrrolidon, N-Methylcaprolactam, Dimethylsulfoxid, Dimethylsulfon und Tetramethylensulfon.

Bevorzugt sind Dimethylacetamid, N-Metylpyrrolidon und Tetramethylensulfon.

Bezogen auf 1 mol eingesetztes Fluorierungsmittel kann man beispielsweise von 50 bis 200 ml eines Lösungsmittels einsetzen. Man kann auch Gemische verschiedener Lösungsmittel verwenden.

Die erfindungsgemäße Fluorierung kann man beispielsweise bei Temperaturen von 160 bis 230°C durchführen. Bevorzugt sind 180 bis 210°C.

Der Druck kann während der Reaktion beispielsweise 1 bis 20 bar betragen. Bevorzugt sind 1 bis 6 bar.

Das erfindungsgemäße Verfahren kann man diskontinuierlich und kontinuierlich durchführen.

Das nach Durchführung des erfindungsgemäßen Verfahrens vorliegende Reaktionsgemisch kann man beispielsweise aufarbeiten, indem man die Reaktionsprodukte durch Destillation abtrennt.

Es ist im allgemeinen vorteilhaft, bei der Durchführung des erfindungsgemäßen Verfahrens zunächst das Fluorierungsmittel und das Lösungsmittel vorzulegen und dann, gegebenenfalls im Vakuum, eine kleine Menge Lösungsmittel zusammen mit eventuell vorhandenem Wasser abzudestillieren und erst dann den Benzaldehyd der Formel (II) und gegebenenfalls einen Katalysator und/oder ein Inertgas (z.B. N₂) zuzugeben.

In das erfindungsgemäße Verfahren einsetzbare Benzaldehyde der Formel (II) kann man beispielsweise aus dem entsprechenden 3-Amino-4-chlortoluol oder 5-Amino-2,4-dichlortoluol herstellen. Diese kann man durch Umsetzung mit Natriumnitrit und Dimethylamin und anschließendem Verkochen in Gegenwart von Fluoridionen in das entsprechende 3-Fluor-chlortoluol überführen (Reaktionsprinzip siehe US-PS 4 075 252).

Aus diesem Fluorchlortoluol kann man durch Chlorierung der Methylgruppe und anschließender saurer Verseifung den entsprechenden Benzaldehyd der Formel (II) erhalten (Reaktionsprinzip siehe Houben-Weyl, Methoden der organischen Chemie, 4. Auflage, Band VII/1, S. 211 ff.)

Dieser Zugang zu den benötigten Benzaldehyden der Formel (II) wird durch folgendes Formelschema beispielhaft illustriert:

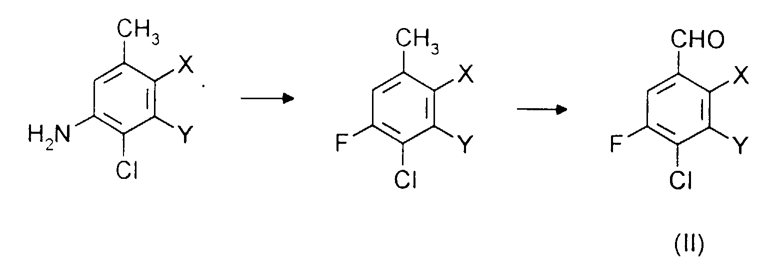

Die in diesem Fall als Startmaterialien benötigten 3-Amino-4-chlortoluole sind Handelsprodukte oder können durch Kernchlorierung aus bekannten Säurehalogeniden über eine Rosenmund-Reaktion erhalten werden.

Die gemäß dem erfindungsgemäßen Verfahren herstellbaren kernfluorierten Benzaldehyde der Formel (I) sind wichtige Zwischenprodukte zur Herstellung von Arzneimitteln.

Man kann aus den kernfluorierten Benzaldehyden der Formel (I) beispielsweise antibakterielle Mittel herstellen, indem man sie zunächst durch Chlorierung an der Aldehydgruppe in das entsprechende Benzoylchlorid überführt. Dieses Benzoylchlorid kann man dann gemäß der EP-A1-550 903 zu einem antibakteriellen Mittel umsetzen.

Im Hinblick auf den eingangs geschilderten Stand der Technik ist es ausgesprochen überraschend, daß es erfindungsgemäß gelingt, bei einem 3-Fluor-4-chlorbenzaldehyd das Chlor gegen Fluor auszutauschen. Weiterhin ist überraschend, daß man je nach der gewählten Menge des Fluorierungsmittels ein gegebenenfalls in 2-Position zur Aldehydgruppe befindliches Chloratom gegebenenfalls gegen Fluor austauschen kann oder es unverändert belassen kann. Diese Stufenselektivität der Fluorierung war keinesfalls vorhersehbar, da sich beide Chloratome (d.h. die in 2- und 4-Stellung befindlichen) in aktivierten Positionen befinden.

### Beispiele

### Beispiel 1

In einer Rührapparatur wurden 163 g Kaliumfluorid in 500 ml Tetramethylensulfon vorgelegt und im Vakuum andestilliert (Destillat 100 ml). Dann wurden 142 g 2,3,4-Trichlor-5-fluorbenzaldehyd zugegeben und nach 10-stündigem Rühren unter Stickstoff bei 190°C das Produkt unter Reduktion des Druckes bis zu 20 mbar in eine gekühlte Vorlage abdestilliert (120 g Destillat). Die erneute Destillation lieferte 65 g 3-Chlor-2,4,5-trifluorbenzaldehyd (Siedepunkt: 66 bis 68°C/15 mbar) und im Nachlauf 2,3-Dichlor-4,5-difluorbenzaldehyd, der erneut in die Fluorierung eingesetzt werden kann.

### Beispiel 2

In einer Rührapparatur wurden 87 g Kaliumfluorid in 225 ml Tetramethylensulfon vorgelegt und zur Trocknung bei 20 mbar andestilliert. Danach wurden 96,5 g 2,4-Dichlor-5-fluorbenzaldehyd zugegeben und 10 Stunden auf 190°C unter Rühren erhitzt. Das Reaktionsgemisch wurde anschließend bei 20 mbar destilliert. Es wurden 78 g Rohdestillat enthalten, das 22,2 Gew.-% 2,4,5-Trifluorbenzaldehyd und 49,4 Gew.-% 2-Chlor-4,5-difluorbenzaldehyd enthielt.

### Beispiel 3

### Herstellung von Ausgangsmaterial

a) Zu einem Gemisch aus 400 ml 35 Gew.-%iger wäßriger Salzsäure und 1.150 ml Wasser wurden 358 g 3-Amino-4,6-dichlortoluol getropft. Es wurde 1 Stunde bei 50°C gerührt und dann auf 0°C abgekühlt. Danach wurden zwischen 0 und 5°C rasch 168 g Natriumnitrit gelöst in 320 ml Wasser zudosiert und nach der Zugabe noch 30 Minuten lang gerührt.
b) In einem anderen Reaktionsgefäß wurden inzwischen 2.200 ml Wasser und 264 g 40 Gew.-%ige wäßrige Dimethylaminlösung vorgelegt, darin 641 g Soda aufgelöst und 920 ml Chloroform hinzugefügt. Anschließend wurde bei 5°C die gemäß a) erhaltenen Diazoniumsalz-Lösung zudosiert und nach dem Ende der Zugabe 5 Stunden nachgerührt. Anschließend wurden die Phasen getrennt, die organische Phase zweimal mit Wasser gewaschen und nach Abziehen des Lösungsmittels bei vermindertem Druck 420 g rohes 3-Dimethyltriazenyl-4,6-dichlortoluol mit einem Schmelzpunkt von 38 bis 40°C erhalten, das ohne weitere Reinigung in die nächste Umsetzungsstufe eingesetzt wurde.
c) In einer Edelstahl-Druckapparatur wurden 720 ml wasserfreier Fluorwasserstoff vorgelegt und bei -6°C die gemäß b) erhaltene Triazenverbindung zudosiert. Anschließend wurden 8 bar Stickstoff aufgedrückt und unter Rühren aufgeheizt. Bei 130 bis 140°C wurde mit einem Druckhalteventil der Druck auf 20 bar einjustiert. Nach Beendigung der Reaktion (keine weitere Stickstoffentwicklung) wurde das Gemisch abgekühlt und der noch vorhandene Fluorwasserstoff abdestilliert. Der verbleibende Rückstand wurde in Dichlormethan gelöst, die Lösung mit Wasser gewaschen und anschließend das Lösungsmittel im Vakuum entfernt. Aus dem dann verbliebenen Rückstand wurden 397 g 3-Fluor-4,6-dichlortoluol mit einem Siedepunkt von 75°C/15 mbar abdestilliert.
d) 537 g des Produkts aus c) wurden in einer Chlorierungsapparatur aus Glas bei 120°C vorgelegt und mit einer UV-Lampe bestrahlt. Dann wurde elementares Chlor in dem Maße eingeleitet, wie es absorbiert wurde. Sobald das Reaktionsgemisch einen Brechungsindex von n_{D}²⁰ von 1,5500 aufwies, wurde die Chloreinleitung abgebrochen. Das Reaktionsgemisch wurde mit Stickstoff ausgeblasen und über eine 60 cm-Füllkörperkolonne destilliert. Es fielen 340 g 3-Fluor-4,6-dichlorbenzalchlorid mit einem Siedepunkt von 125 bis 126°C/20 mbar an. Der Vorlauf konnte erneut in diese Chlorierung eingesetzt werden.
e) In einer Rührapparatur wurden 600 ml 95 Gew.-%ige Schwefelsäure bei 40°C vorgelegt und 266 g des gemäß d) erhaltenen Benzalchlorids zudosiert. Es wurde bis zum Ende der Gasentwicklung nachgerührt. Dann wurde der Ansatz auf 1 kg Eis ausgetragen und das Produkt mit Dichlormethan aufgenommen. Die Destillation der organischen Phase lieferte 155 g 3-Fluor-4,6-Dichlorbenzaldehyd mit einem Siedepunkt von 110 bis 112°C/20 mbar.

### Beispiel 4

### Anderer Weg zu einem Ausgangsprodukt der Formel (II)

In einer Hydrierapparatur wurden 800 ml trockenes Toluol und 250 g 2,3,4-Trichlor-5-fluorbenzoylchlorid zusammen mit 40 g eines Katalysators vorgelegt, der 5 Gew.-% metallisches Palladium auf einem Bariumsulfat-Träger enthielt. Danach wurde bei 40 bis 80°C Wasserstoff eingeleitet. Der Fortschritt der Hydrierung wurde mittels gaschromatographischer Kontrolle verfolgt und nach dem quantitativen Verbrauch des eingesetzten Benzoylchlorids abgebrochen. Nach Filtration des Reaktionsgemisches wurde die Lösung vom Toluol durch Destillation befreit und der Rückstand aus 223 g Cyclohexan umkristallisiert. Es wurden 148 g 2,3,4-Trichlor-5-fluorbenzaldehyd mit einem Schmelzpunkt von 78 bis 79°C erhalten.

### Beispiel 5

### Umsetzung eines kernfluorierten Benzaldehyds der Formel (I) zu dem entsprechenden Benzoylchlorid

In einer Chlorierungsapparatur mit UV-Bestrahlung wurden 100 ml 2-Chlorbenzotrifluorid und 4,5 g Kaliumchlorid bei 110°C vorgelegt und anschließend 50 g 3-Chlor 3-Chlor-2,4,5-trifluorbenzaldehyd in 50 ml 2-Chlorbenzotrifluorid unter Luftausschluß getropft. Nach Zugabe von 10 ml der Lösung wurde Chlor in dem Maße hinzugefügt, wie es aufgenommen wurde. Die Dosierung wurde solange fortgesetzt, bis die gesamte Menge des eingesetzten Aldehyds umgesetzt war (gaschromatographische Kontrolle). Die Destillation des Reaktionsgemisches lieferte 52 g 3-Chlor-2,4,5-trifluorbenzoylchlorid mit einem Siedepunkt von 92°C/20 mbar.

Beim Einsatz von 2,4,5-Trifluorbenzaldehyd anstelle von 3-Chlor-2,4,5-trifluorbenzaldehyd wurde in ganz analoger Weise 2,4,5-Trifluorbenzoylchlorid erhalten.

## Patentansprüche

1. Verfahren zur Herstellung von 4,5-Difluorbenzaldehyden der Formel (I) 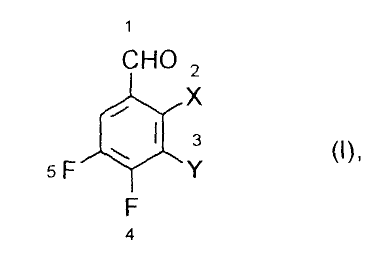 in der
X für Fluor oder Chlor und
Y für Wasserstoff oder Chlor stehen,
dadurch gekennzeichnet, daß man Benzaldehyde der Formel (II) 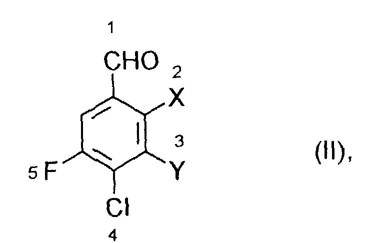 in der aus X und Y die bei Formel (I) angegebene Bedeutung haben, mit einem Fluorierungsmittel umsetzt.

2. Verfahren nach Anspruch 1, dadurch gekennzeichnet, daß man als Fluorierungsmittel Natrium-, Kalium- und/oder Cäsiumfluorid in einer Menge von 0,8 bis 2 mol pro 1 mol auszutauschenden Chlors im eingesetzten Benzaldehyd einsetzt.

3. Verfahren nach Ansprüchen 1 und 2, dadurch gekennzeichnet, daß man es in Gegenwart eines Lösungsmittels durchführt.

4. Verfahren gemäß Ansprüchen 1 bis 3, dadurch gekennzeichnet, daß man es bei Temperaturen im Bereich 160 bis 230°C durchführt.

5. Verfahren nach Ansprüchen 1 bis 4, dadurch gekennzeichnet, daß man Benzaldehyde der Formel (II) einsetzt, die aus dem entsprechenden 3-Amino-4-chlortoluol oder dem entsprechenden 5-Amino-2,4-dichlortoluol durch Umsetzung mit Natriumnitrit und Dimethylamin und anschließendem Verkochen in Gegenwart von Fluordionen und Chlorierung und saurer Verseifung des dabei erhältlichen Fluorchlortoluols erhältlich sind, einsetzt.

## Claims

1. Process for preparing 4,5-difluorobenzaldehydes of the formula (I) 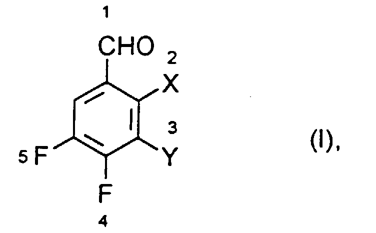 in which
X represents fluorine or chlorine and
Y represents hydrogen or chlorine,
characterized in that benzaldehydes of the formula (II) 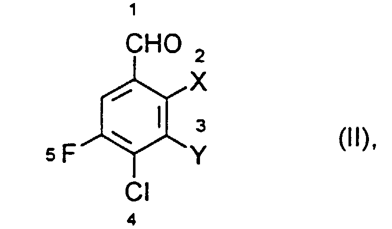 in which X and Y are as defined for formula (I), are reacted with a fluorinating agent.

2. Process according to Claim 2, characterized in that the fluorinating agent used is sodium, potassium and/or caesium fluoride in an amount of from 0.8 to 2 mol per 1 mol of chlorine to be replaced in the benzaldehyde used.

3. Process according to either of Claims 1 and 2, characterized in that it is carried out in the presence of a solvent.

4. Process according to any of Claims 1 to 3, characterized in that it is carried out at temperatures in the range from 160 to 230°C.

5. Process according to any of Claims 1 to 4, characterized in that the benzaldehydes of the formula (II) used are those which are obtainable from the corresponding 3-amino-4-chlorotoluene or the corresponding 5-amino-2,4-dichlorotoluene by reaction with sodium nitrite and dimethylamine and subsequent boiling down in the presence of fluoride ions, and chlorination and acid saponification of the fluorochlorotoluene obtainable in this way.

## Revendications

1. Procédé pour la préparation de 4,5-difluorobenzaldéhydes de la formule (I) 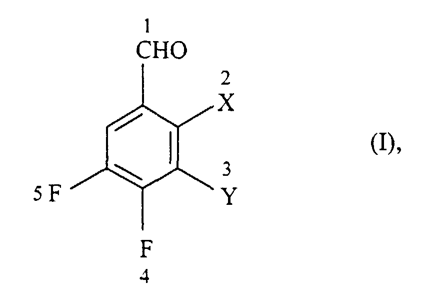 dans laquelle
X représente le fluor ou le chlore et
Y représente l'hydrogène ou le chlore,
caractérisé en ce que l'on transforme des benzaldéhydes de la formule (II) 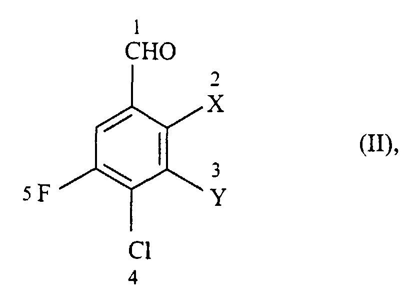 dans laquelle X et Y ont la signification indiquée dans la formule (I) avec un agent de fluoration.

2. Procédé selon la revendication 1, caractérisé en ce que l'on utilise comme agent de fluoration du fluorure de sodium, de potassium et/ou de césium dans une quantité de 0,8 à 2 mol pour une mole de chlore à échanger dans le benzaldéhyde utilisé.

3. Procédé selon les revendications 1 et 2, caractérisé en ce qu'il est réalisé en présence d'un solvant.

4. Procédé selon les revendications 1 à 3, caractérisé en ce qu'il est réalisé à des températures dans le domaine de 160 à 230°C.

5. Procédé selon les revendications 1 à 4, caractérisé en ce que l'on utilise des benzaldéhydes de la formule (II) qui sont obtenus à partir du 3-amino-4-chlorotoluène correspondant ou du 5-amino-2,4-dichlorotoluène correspondant par réaction avec du nitrite de sodium et de la diméthylamine et par transformation par ébullition subséquente en présence d'ions fluor et par chloration et saponification acide du fluorochlorotoluène ce faisant obtenu.
